# DEMANDE DE BREVET EUROPEEN

(11) **EP 1 247 571 A1**
(43) Date de publication de la demande: **09.10.2002**
(21) Numéro de dépôt: 02076349.6
(22) Date de dépôt: 04.04.2002
(51) Int. Cl.: B01J 20/28, A01N 25/18, A61L 9/04, A61K 7/46, B01J 20/24, B65D 65/42, B65D 81/28, B29C 70/10, C08J 3/20

(54) **Nouveaux éléments actifs contenant des fibres, leur procédé de fabrication, et leurs applications notammment dans l'emballage**

(30) Priorité: 06.04.2001 FR 0104918
(71) Demandeur: AFT Plasturgie, 21121 Fontaine les Dijon (FR)
(72) Inventeur: Mougin, Gerard, 21190 Meursault (FR)
(74) Mandataire: Richebourg, Michel François

(57) **Abrégé**

Matériau apte à former des éléments de forme et destination quelconques, notamment, mais à titre non limitatif, pour emballages, supports olfactifs, conditionnements industriels, matériaux de décoration, absorbeurs d'odeurs ou d'humidité ou d'autres substances, et produits analogues, dans lequel on introduit au moins un support absorbant du type poreux apte à créer des cavités s'ouvrant au moins en partie à la surface dudit matériau, cavités qui sont aptes à accueillir une quantité efficace de principe actif par absorption et à relarguer une dose efficace et sensiblement régulière, dans le temps, dudit principe actif. Ledit matériau est constitué, en ce qui conceme sa matrice, d'une matière plastique ou d'un mélange de telles matières plastiques, notamment des polymères ou copolymères. Parmi les supports absorbants susceptibles de convenir, on trouve les fibres naturelles. Emballages entre autres avec les fonctions suivantes protection contre les bactéries, les acariens, les insectes, les moisissures, accélération ou ralentissement du mûrissement , absorption ou émission de gaz fonctionnels, maintien d'une atmosphère particulière (ex : humidité), absorption de l'humidité (emploi d'un superabsorbant pour mouiller les fibres),...

## Description

### Secteur technique de l'invention :

La présente invention concerne le secteur technique des produits et éléments pouvant être utilisés comme conditionnements ou emballages, ci après ensemble et par simplicité, y compris dans les revendications, « emballages ».

L'invention concerne plus particulièrement le secteur technique des éléments ou produits de ce type (ci après ensemble, par simplicité, y compris dans les revendications, « éléments ») présentant des fonctions de type « actif » ou « intelligent » comme cela est expliqué ci-dessous.

### Problème technique posé :

Dans l'industrie alimentaire, les matériaux pour l'emballage ont connu ces dernières années une évolution exponentielle en termes de solutions apportées à la conservation des denrées périssables. Parmi les innovations récentes, on constate un intérêt croissant pour la fabrication d'emballages dits « intelligents ». Il s'agit de produits destinés à contenir des denrées périssables, et on associe à ces produits une ou plusieurs fonctionnalités. Pour cela le matériau constitutif présente une réaction prédéterminée face à une variation de l'état de l'environnement du produit emballé. Comme exemple, on peut citer les films d'emballage qui absorbent l'oxygène pouvant entrer accidentellement en contact avec le produit, afin de préserver celui-ci d'une oxydation trop rapide. D'autres fonctions similaires sont réalisées; comme le relargage par le film d'emballage de composés bactéricides prévenant ainsi une dégradation.

Les difficultés majeures freinant l'imagination des concepteurs résident dans les points suivants :
- Les produits actifs que doivent diffuser les emballages sont souvent des molécules organiques qui se dégradent facilement aux températures de mise en oeuvre des polymères. Il est donc difficile de les incorporer au cours de la production de l'emballage lui-même.
- La vitesse de diffusion est généralement mal maîtrisée, de même que la concentration du produit relargué.

Un objet de l'invention est de proposer des éléments, notamment en matière synthétique ou plastique, comportant de telles fonctionnalités.

Les éléments visés doivent présenter les propriétés suivantes :
. capacité à incorporer et diffuser une ( ou plusieurs ) substance active par rétention ou re largage d'un composé dont la stabilité thermique est pourtant faible.
. possibilité d'utiliser la totalité de la substance active ou du principe actif et de recharger le produit de nombreuses fois pour des usages successifs identiques ou non (changement ou non de principe actif).
. maîtrise de la quantité et de la vitesse de re largage du principe actif.
. recyclabilité totale du produit.

### Art antérieur :

En dehors des fonctions de forme, de couleur et de tenue mécanique, apportées de façon classique aux objets en matières plastiques , d'autres sont plus spécifiques comme par exemple la résistance aux UV ou au feu. Ces fonctions sont obtenues par incorporation dans le polymère d'un ou plusieurs produits chimiques devant changer son comportement vis à vis d'un changement de son environnement. Dans tous les cas cités, le comportement de l'objet est « statique » et l'additif incorporé n'agit qu'en cas de sollicitation externe.

Il existe quelques cas dans lesquels la réaction de l'objet sur l'environnement est spontanée, et on peut citer comme exemple la diffusion de parfums, d'insecticides... par des supports plastiques imbibés de produits actifs lors de leur fabrication . Dans ce cas, la diffusion du produit n'est que très mal contrôlée et, compte tenu des effets de concentration en surface, elle varie de façon importante dans le temps.

Dans tous les cas, les additifs actifs sont ajoutés au polymère lors de la fabrication de la matière ou au mieux de l'objet, c'est à dire que le produit doit être stable aux températures de transformation du polymère. Ce fait limite la technique à l'emploi de produits minéraux ou organiques peu mobiles donc à faible effet de diffusion. En outre, on est obligé de traiter la totalité du polymère, donc de l'objet, ce qui entraîne la présence de principe actif au coeur de l'épaisseur, produit qui restera donc définitivement inutilisé du fait de sa non disponibilité en surface ou à proximité suffisante de la surface.

Un autre inconvénient majeur de la technique actuelle réside dans le fait que les objets deviennent définitivement inactifs après épuisement du produit diffusant. De plus la teneur résiduelle en principe actif resté piégé au coeur de la matière, comme on vient de le voir, pose des problèmes écologiques pour le recyclage éventuel de l'objet, sans compter naturellement la perte matière de principe actif généralement onéreux.

On a déjà essayé de contourner ces inconvénients en déposant sur la surface de l'objet le principe actif, soit directement soit à l'état imbibé ou incorporé dans un support. Malheureusement cette technique n'est pas fiable car le moindre frottement avec la surface entraîne une consommation accidentelle du produit actif.

II existe donc un besoin important et reconnu pour un matériau capable de former des éléments diffuseurs dans lesquels on pourrait facilement incorporer au moins un principe actif (ou une substance précurseur de ce principe actif, ci après, , y compris dans les revendications, « principe actif », le singulier englobant aussi le pluriel, comme le comprendra aisément l'homme de métier), à partir duquel ledit principe actif pourrait diffuser de manière contrôlée en fonction du temps, en totalité ou essentiellement en totalité (c'est-à-dire avec très peu de principe actif résiduel dans la matrice de matériau, lorsque la fonctionnalité du principe actif a atteint un niveau tel que l'on doit remplacer l'élément diffuseur ou le recharger, comme on le verra ci-dessous), que l'on puisse de préférence recharger sans difficultés, et qui enfin n'impose pas au principe actif des contraintes dénaturantes, comme des contraintes thermiques ou autres.

### Résumé de l'invention :

Dans son aspect le plus général, la présente invention est caractérisée par un matériau (ce vocable incluant toute structure , simple ou composite, apte à former ou générer lesdits éléments décrits ci-après) apte à former des éléments de forme et destination quelconques, notamment, mais à titre non limitatif, pour emballages, supports olfactifs, conditionnements industriels, matériaux de décoration, absorbeurs d'odeurs ou d'humidité ou d'autres substances, et produits analogues, dans lequel on introduit au moins un support absorbant du type poreux apte à créer des cavités s'ouvrant au moins en partie à la surface dudit matériau, cavités qui sont aptes à accueillir une quantité efficace de principe actif par absorption et à relarguer une dose efficace et sensiblement régulière, dans le temps, dudit principe actif.

Il est rappelé que « principe actif» couvre, y compris dans les revendications, non seulement le principe actif lui-même, mais également un précurseur de ce principe actif, ou éventuellement un produit ou substance lui-même capable de relarguer ledit principe actif, ainsi que les mélanges, associations, combinaisons, de tels « principes actifs », de même fonctionnalité (par exemple combinaison de parfums) ou de fonctionnalités différentes (par exemple un parfum et une substance déodorante ou anti-acariene, etc...) et variantes analogues qui apparaîtront clairement à l'homme de métier sans qu'il soit nécessaire de le préciser à nouveau.

L'invention concerne également le procédé de fabrication de ce matériau, ainsi que ses applications, et concerne encore les « éléments » formés en ledit matériau (ou comprenant au moins en partie ledit matériau), ainsi que les applications de ces éléments, déjà citées ou suggérées ci-dessus.

Cette solution n'a jamais été envisagée, peut être en raison des préjugés évidents suivants :
- le surcoût car il faut ajouter un produit dans toute la masse du matériau,
- risque que les pores soient complètement bouchés par la matière plastique lors de toute transformation.
- risque qu'une trop faible proportion de « cavités » s'ouvre sur la surface.
- risque d'affaiblir les propriétés mécaniques de la matrice de par la présence d'hétérogénéités dans sa masse.
- obligation de disposer d'un volume « réservoir » important, et donc d' une grande quantité d'absorbant, avec un risque accru de dégrader les propriétés du matériau.
- impossibilité de prévoir les caractéristiques de diffusion, notamment une diffusion régulière et reproductible.

Selon l'invention, on a montré que l'on pouvait résoudre tous les problèmes de l'art antérieur, et contourner les préjugés qui semblaient insurmontables, par un procédé selon lequel on utilise pour fabriquer des « éléments », ou on incorpore dans ces « éléments », et ce dès la fabrication desdits « éléments », notamment des emballages, un matériau absorbant et relarguant un principe actif, ledit principe actif étant lui-même ajouté lors d'une étape ultérieure de fabrication ou lors de l'utilisation.

### Description détaillée de l'invention :

Selon un mode de réalisation préféré, ledit matériau est constitué, en ce qui concerne sa matrice, d'une matière plastique ou d'un mélange de telles matières plastiques, notamment des polymères ou copolymères.

A titre non limitatif de telle matrice, on citera tous les (co)polymères aptes à former des films souples ou à être autrement mis en forme, comme sous la forme de plaques, surfaces planes ou non.

Tous les polymères peuvent convenir, qu'ils soient thermoplastiques ou thermodurcissables. Les thermoplastiques dits de commodité (Polyéthylène, Polystyrène, Polypropylène) et les polyesters insaturés sont particulièrement adaptés en raison de leurs conditions de transformation, notamment température relativement basse de transformation, préservant l'intégrité du support absorbant, notamment des fibres naturelles, mais aussi du fait qu'ils sont les matériaux les plus utilisés dans les applications visées.

On citera notamment les (co)polymères suivants :
- polyoléfines : polyéthylènes, polypropylènes, polyisobutylène, polyméthylpentène,
- polychlorures de vinyle
- polyacétates de vinyle et acétates polyvinyliques
- polymères styréniques: PS (polystyrène), ABS (acrylonitrile butadiène styrène), SB (styrène-butadiène), SAN (styrène acrylonitrile), ASA (acrylonitrile styrène acrylates)
- polyacétals : POM ( polyoxyméthylène)
- polyesters saturés et insaturés
- polyépoxydes
- aminoplastes : MF (mélamineformaldéhyde), UF (urée formaldéhyde)
- polymères phénoliques
- silicones
- polyuréthanes
- polyamides : 6, 6-6, 4-6, 6-12, 6-10, 11, 12 et aromatiques
- polymères acryliques
- polymères cellulosiques
- polycarbonates
- polymères fluorés: PVDF (fluorure de vinylidène), PEP ( (polyéthylènepropylène) perfluoré), ETFE (ethylène tétrafluoroéthylène), PCTFE (polychlorotrifluoroéthylène)
et analogues bien connus de l'homme de métier,
et de manière générale tout matériau pouvant être mis en forme à une température inférieure à 350 °C environ, et susceptible de voir ses propriétés renforcées par l'addition de fibres naturelles: béton, bitume, métaux à bas point de fusion, poudres céramiques comprimées, polymères naturels comme amidon, cellulose, et leurs dérivés et analogues connus de l'homme du métier.

Parmi les supports absorbants susceptibles de convenir, on trouve les fibres naturelles. Celles-ci peuvent être en effet incorporées aux polymères afin par exemple de renforcer leurs propriétés mécaniques. L'objet fabriqué en polymère présente alors une surface au niveau de laquelle débouchent un certain nombre des fibres incorporées. De par leur structure tubulaire, les fibres sont capables d'absorber et de stocker une quantité importante d'un fluide quelconque.

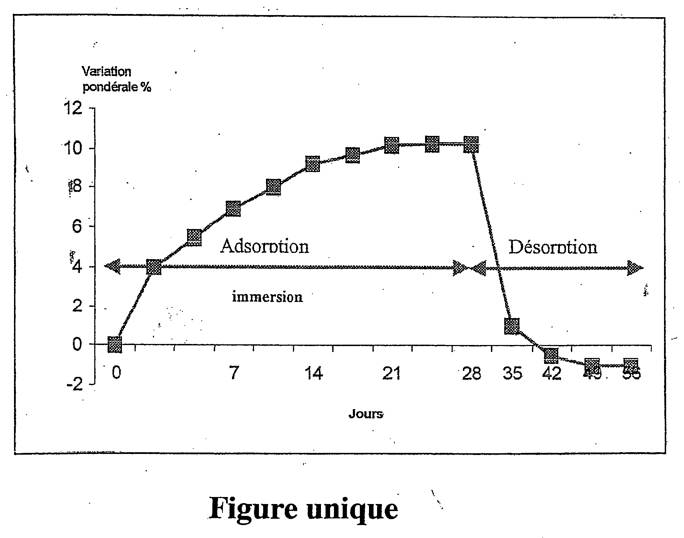

A titre d'exemple, on a représenté sur la figure unique annexée un cycle d'absorption et de désorption d'eau par une plaque de polyester insaturé contenant des fibres de chanvre à raison de 30 % en poids . La plaque est d'abord plongée dans l'eau puis laissée à l'air ambiant (23 °C et 93 % HR). L'homme de métier comprendra que la partie intéressante du cycle concerne les premiers jours pendant lesquels la pente de la courbe est maximale et pratiquement constante. Lorsque le palier est atteint ( après environ 15 j dans ce cas non limitatif), la vitesse de diffusion devient trop faible pour présenter un intérêt. Dans le cas d'une nécessité de protection sur une durée plus courte ou plus longue, l'homme de métier saura envisager une modification de la vitesse de diffusion par des altérations de la viscosité, tension superficielle, et autres paramètres connus du fluide, et / ou la fermeture de l'emballage.

Toutes les fibres naturelles sont envisageables : chanvre, lin, sisal, , pailles, jute, coton, bois... mais on peut utiliser aussi des produits poreux quelconques comme par exemple la terre de diatomées ou des sphères de verre broyées qui en tant que charge minérale présente l'avantage de supporter de hautes températures.

Les fibres naturelles peuvent être utilisées sous diverses formes géométriques, dans les applications thermoplastiques on les choisira d'une longueur inférieure à 30 ou 40 mm, de préférence inférieure à 20 mm afin de ne pas perturber les procédés d'injection ou d'extrusion. Leur concentration sera ajustée par l'homme de métier en fonction des propriétés mécaniques à atteindre et de la quantité réserve de principe actif à incorporer, de ses connaissances générales et de quelques essais simples de routine, éventuellement.

L'utilisation comme absorbant de fibres naturelles permet de résoudre tous les problèmes précités :
. les fibres sont des additifs de renforcement , et leur introduction dans les plastiques améliore les propriétés mécaniques de ceux ci. Elles sont d'un coût modeste et, étant de faible densité (deux fois plus faible que le verre), un petit pourcentage pondéral occupe un grand volume.
. les fibres ont une structure creuse et avec les procédés de transformation on obtient une quantité reproductible de fibres émergeant en surface, cette quantité étant proportionnelle ou sensiblement proportionnelle à la quantité totale de fibres introduites.
. les fibres émergeant en surface se comportent comme des capillaires capables d'accueillir un fluide. Les dimensions des cavités ou pores ainsi créés sont suffisantes pour permettre au principe absorbé d'être relargué par le simple gradient de concentration avec l'extérieur. Il est à noter que dans le cas du verre les espaces capillaires entre les fibres et la matrice polymère permettent l'adsorption d'un fluide mais pas sa diffusion compte tenu de la faible dimension des cavités.
. le phénomène d'absorption / désorption d'un fluide quelconque est reproductible indéfiniment.
. les fibres peuvent être entièrement ou sensiblement entièrement débarrassées (à la désorption) du principe actif absorbé, autorisant ainsi une optimisation de l'emploi du principe actif ainsi qu'un recyclage du matériau ou de l'élément (il suffit de recharger l'élément en principe actif) ou sa réutilisation dans une autre fonction apportée par un principe actif différent. De même on peut utiliser simultanément plusieurs composés ou principes actifs diffusants. Dans tous les cas les composés n'ont pas à avoir d'affinité ou de compatibilité avec la matrice de polymère car ils n'entrent pas à son contact, puisqu'ils sont confinés dans la fibre.

On emploiera naturellement, cependant, des principes actifs compatibles avec la matrice.

On peut envisager aussi de remplir les capillaires (des fibres incorporées) avec un fluide dont la viscosité ou la tension superficielle ou tout autre paramètre interdise sa diffusion ultérieure. On obtient ainsi un produit fonctionnalisé postérieurement à sa fabrication, donc la possibilité d'utiliser un agent à très faible stabilité thermique.
Plusieurs paramètres de l'incorporation sont aisément modulables, notamment si l'on envisage comme principe actif un fluide à base de solvant contenant ledit principe actif :
- En variant la nature du fluide (sa viscosité ou son affinité pour la fibre entre autres) on va faire varier la vitesse de relargage.
- En faisant varier la concentration de principe actif dans la fibre, on fera varier de même la concentration en produit diffusé.
- En faisant varier la teneur en fibres du support polymère, on fera varier aussi la concentration du diffusant, mais on aura aussi une quantité de principe actif disponible plus ou moins grande.
- On peut enfin faire varier de la même manière la quantité globale de principe actif à diffuser en laissant le matériau immergé plus ou moins longtemps dans le fluide.

La fabrication d'éléments de toutes sortes en polymères (ou autre type de matrice) chargés de fibres naturelles permet donc la réalisation de produits industriels fonctionnalisés, et notamment d'emballages fonctionnels. Il suffit d'immerger le matériau, ou l'élément complet, dans un principe actif ou dans un fluide contenant ledit principe actif, ou de traiter le produit industriel fini, notamment l'emballage, pour que ce dernier devienne multi-fonctionnel. La liste des fonctions apportées à l'emballage par les substances actives n'est pas limitée, non plus que la liste des applications.
Dans le domaine alimentaire, qui représente un potentiel important bien que n'étant pas la seule application, on peut envisager de créer des emballages entre autres avec les fonctions suivantes :
- protection contre les bactéries, les acariens, les insectes, les moisissures ...
- accélération ou ralentissement du mûrissement ...
- absorption ou émission de gaz fonctionnels ...
- maintien d'une atmosphère particulière (ex : humidité)
- absorption de l'humidité (emploi d'un superabsorbant pour mouiller les fibres), etc....

Un des avantages majeurs du procédé est la possibilité de répétition du phénomène donc de recirculation ou recyclage des éléments, notamment des emballages, la fonctionnalité étant en quelque sorte « régénérée » par un nouveau traitement de l'élément. On peut même envisager que le même élément, notamment un emballage, puisse dans des étapes successives remplir des fonctions différentes grâce au traitement par des substances actives différentes.

Enfin une immersion adaptée dans un solvant neutre permet de supprimer toute trace de substance active, donc de régénérer un élément, notamment un emballage, inactif et donc prêt pour une nouvelle utilisation.

Selon un mode de réalisation préféré, la matrice chargée en fibres, de préférence naturelles, doit contenir au moins 5 à 10 % en poids, ou mieux au moins 20% de matrice, notamment de matrice polymère.

En ce qui concerne le cas d'une matrice polymère, et quel que soit le type de (co)polymère utilisé, les procédés classiques de « compoundage » (c'est-à-dire de mélange de la charge fibreuse avec le polymère ) peuvent être utilisés pour l'incorporation des fibres, et ne présentent pas de difficulté particulière.

Le principe actif peut être introduit sous forme liquide ou même gazeuse ou encore solide, et dans ces deux derniers cas il est préférable d'utiliser une solution liquide du ou des principes actifs.

Le liquide aura des caractéristiques (viscosité, tension superficielle, concentration...) qui permettent d'ajuster sa vitesse de diffusion. Il pourra être introduit dans les porosités de l'objet par tout moyen comme le trempé , la pulvérisation, l'essuyage ....

Ces procédés peuvent être éventuellement mis en oeuvre à des pressions et températures différentes des conditions standard (# 20° C, 1 atm) afin de faciliter la pénétration par absorption et / ou capillarité.

On peut envisager de traiter de la manière décrite ci dessus l'ensemble de l'élément ou certaines parties seulement, en utilisant les techniques habituelles de la plasturgie pour la réalisation d'objets multimatières.

Les applications de l'invention sont extrêmement nombreuses, à titre d'exemples :
. Emballages agro-alimentaires: diffusion de produits bactéricides, fongicides, insecticides, d'agents de mûrissement, d'atmosphères protectrices, absorption ou désorption d'eau...
. Conditionnements industriels: relargage de produits de protection, antirouille, contrôle de l'humidité..
. Produits grand public : pièces utilitaires ou simples supports pour la diffusion d'agents antibactérien, antiacarien, antifongique..., insecticide, déodorant, désinfectant, odorant, parfum, ..., comme filtres pour aspirateurs, éléments de systèmes de climatisation et traitement de l'air du bâtiment ou des transports, poubelles ou sacs poubelle, supports olfactifs et objets de « marketing olfactif » comme des jouets olfactifs, matériaux de décoration parfumés ou de contrôle de l'humidité, ou insecticides, déodorants, anti-tabac,
. Emballages divers: contrôle de l'humidité, absorbeurs d'oxygène, diffusion de produits bactéricides, fongicides, insecticides, d'agents de mûrissement, d'atmosphères protectrice,...

Il est rappelé que « principe actif » couvre, y compris dans les revendications, non seulement le principe actif lui-même, mais également un précurseur de ce principe actif, ou éventuellement un produit ou substance lui-même capable de relarguer ledit principe actif, ainsi que les mélanges, associations, combinaisons, de tels « principes actifs », de même fonctionnalité (par exemple combinaison de parfums) ou de fonctionnalités différentes (par exemple un parfum et une substance déodorante ou anti-acarienne, etc...) et variantes analogues qui apparaîtront clairement à l'homme de métier sans qu'il soit nécessaire de le préciser à nouveau.

L'invention couvre également tous les modes de réalisation et toutes les applications qui seront directement accessibles à l'homme de métier à la lecture de la présente demande, de ses connaissances propres, et éventuellement d'essais simples de routine.

## Revendications

1. Matériau ,ce vocable incluant toute structure, simple ou composite, apte à former ou générer lesdits éléments décrits ci-après, apte à former des éléments de forme et destination quelconques, notamment, mais à titre non limitatif, pour emballages, supports olfactifs, conditionnements industriels, matériaux de décoration, absorbeurs d'odeurs ou d'humidité ou d'autres substances, et produits analogues, **caractérisé en ce que** il consiste en une matrice dans laquelle on introduit ou charge au moins un support absorbant du type poreux apte à créer des cavités s'ouvrant au moins en partie à la surface dudit matériau, cavités qui sont aptes à accueillir une quantité efficace de principe actif par absorption et à relarguer une dose efficace et sensiblement régulière, dans le temps, dudit principe actif.

2. Matériau selon la revendication 1 **caractérisé en ce que** le « principe actif » est choisi parmi le principe actif lui-même, un précurseur de ce principe actif, ou un produit ou substance lui-même capable de relarguer ledit principe actif, ainsi que les mélanges, associations, combinaisons, de tels « principes actifs », de même fonctionnalité comme des combinaison de parfums ou de fonctionnalités différentes comme un parfum et une substance déodorante ou anti-acarienne.

3. Matériau selon la revendication 1 ou 2 **caractérisé en ce que** sa matrice est constituée d'une matière plastique ou d'un mélange de telles matières plastiques, notamment des polymères ou copolymères, dont tous les (co)polymères aptes à former des films souples ou à être autrement mis en forme, comme sous la forme de plaques, surfaces planes ou non, et **en ce que** de préférence la matrice chargée confient au moins 5 à 10 % en poids, ou mieux au moins 20% de matrice, notamment de matrice polymère.

4. Matériau selon la revendication 3 **caractérisé en ce que** lesdits polymères sont thermoplastiques ou thermodurcissables, comme les thermoplastiques dits de commodité (Polyéthylène, Polystyrène, Polypropylène) et les polyesters insaturés, et sont notamment choisis parmi les suivants :
- polyoléfines : polyéthylènes, polypropylènes, polyisobutylène, polyméthylpentène,
- polychlorures de vinyle
- polyacétates de vinyle et acétates polyvinyliques
- polymères styréniques : PS (polystyrène), ABS (acrylonitrile butadiène styrène), SB (styrène-butadiène), SAN (styrène acrylonitrile), ASA (acrylonitrile styrène acrylates)
- polyacétals : POM ( polyoxyméthylène)
- polyesters saturés et insaturés
- polyépoxydes
- aminoplastes : MF (mélamine formaldéhyde), UF (urée formaldéhyde)
- polymères phénoliques
- silicones
- polyuréthanes
- polyamides : 6, 6-6, 4-6, 6-12, 6-10, 11, 12 et aromatiques
- polymères acryliques
- polymères cellulosiques
- polycarbonates
- polymères fluorés: PVDF (fluorure de vinylidène), PEP ( (polyéthylènepropylène) perfluoré), ETFE (éthylène tétrafluoroéthylène) , PCTFE (polychlorotrifluoroéthylène)
et de manière générale tout matériau pouvant être mis en forme à une température inférieure à 350 °C environ, et susceptible de voir ses propriétés renforcées par l'addition de fibres naturelles: béton, bitume, métaux à bas point de fusion, poudres céramiques comprimées, polymères naturels comme amidon, cellulose.

5. Matériau selon l'une quelconque des revendications 1 à 4 **caractérisé en ce que** les supports absorbants sont les fibres naturelles ou des produits poreux quelconques comme la terre de diatomées ou des sphères de verre broyées.

6. Matériau selon la revendication 5 **caractérisé en ce que** les fibres sont choisies parmi : chanvre, lin, sisal, kenaf, jute, coton, bois ,pailles.

7. Matériau selon la revendication 5 ou 6 **caractérisé en ce que** les fibres naturelles sont utilisées sous diverses formes géométriques comme, dans les applications thermoplastiques, d'une longueur inférieure à 30 ou 40 mm, de préférence inférieure à 20 mm.

8. Procédé pour fabriquer des matériaux selon l'une quelconque des revendications 1 à 7, **caractérisé en ce qu'** on incorpore dans une matrice au moins un support absorbant du type poreux apte à créer des cavités s'ouvrant au moins en partie à la surface dudit matériau, cavités qui sont aptes à accueillir une quantité efficace de principe actif par absorption et à relarguer une dose efficace et sensiblement régulière, dans le temps, dudit principe actif.

9. Procédé selon la revendication 8 **caractérisé en ce que** la matrice est constituée d'une matière plastique ou d'un mélange de telles matières plastiques, notamment des polymères ou copolymères, dont tous les (co)polymères aptes à former des films souples ou à être autrement mis en forme, comme sous la forme de plaques, surfaces planes ou non.

10. Procédé selon la revendication 9 **caractérisé en ce que** lesdits polymères sont thermoplastiques ou thermodurcissables, comme les thermoplastiques dits de commodité (Polyéthylène, Polystyrène, Polypropylène) et les polyesters insaturés, et sont notamment choisis parmi les suivants :
- polyoléfines : polyéthylènes, polypropylènes, polyisobutylène, polyméthylpentène,
- polychlorures de vinyle
- polyacétates de vinyle et acétates polyvinyliques
- polymères styréniques: PS (polystyrène), ABS (acrylonitrile butadiène styrène), SB (styrène-butadiène), SAN (styrène acrylonitrile), ASA (acrylonitrile styrène acrylates)
- polyacétals : POM ( polyoxyméthylène)
- polyesters saturés et insaturés
- polyépoxydes
- aminoplastes : MF (mélamine formaldéhyde), UF (urée formaldéhyde)
- polymères phénoliques
- silicones
- polyuréthanes
- polyamides : 6, 6-6, 4-6, 6-12, 6-10, 11, 12 et aromatiques
- polymères acryliques
- polymères cellulosiques
- polycarbonates
- polymères fluorés: PVDF (fluorure de vinylidène), PEP ( (polyéthylènepropylène) perfluoré), ETFE (éthylène tétrafluoroéthylène) , PCTFE (polychlorotrifluoroéthylène)
et de manière générale tout matériau pouvant être mis en forme à une température inférieure à 350 °C environ, et susceptible de voir ses propriétés renforcées par l'addition de fibres naturelles: béton, bitume, métaux à bas point de fusion, poudres céramiques comprimées, polymères naturels comme amidon, cellulose.

11. Procédé selon l'une quelconque des revendications 8 à 10 **caractérisé en ce que** les supports absorbants sont les fibres naturelles ou des produits poreux quelconques comme par exemple la terre de diatomées ou des sphères de verre broyées, **en ce que** les fibres sont choisies parmi : chanvre, lin, sisal, kenaf, jute, coton, bois, pailles, **en ce que** les fibres naturelles sont utilisées sous diverses formes géométriques comme, dans les applications thermoplastiques, d'une longueur inférieure à 30 ou 40 mm, de préférence inférieure à 20 mm.

12. Procédé selon l'une quelconque des revendications 8 à 11 **caractérisé en ce que** les fibres peuvent être entièrement ou sensiblement entièrement débarrassées, à la désorption, du principe actif absorbé, autorisant ainsi une optimisation de l'emploi du principe actif ainsi qu'un recyclage du matériau ou de l'élément ou sa réutilisation dans une autre fonction apportée par un principe actif différent, ou on peut utiliser simultanément plusieurs composés ou principes actifs diffusants, et de préférence des principes actifs compatibles avec la matrice.

13. Procédé selon l'une quelconque des revendications 8 à 12 **caractérisé en ce que** dans le cas d'une matrice polymère, et quel que soit le type de (co)polymère utilisé, les procédés classiques de « compoundage », c'est-à-dire de mélange de la charge fibreuse avec le polymère , peuvent être utilisés pour l'incorporation des fibres ou du matériau poreux.

14. Procédé selon l'une quelconque des revendications 8 à 13 **caractérisé en ce que** le principe actif peut être introduit sous forme liquide ou même gazeuse ou encore solide, et dans ces deux damiers cas il est préférable d'utiliser une solution liquide du ou des principes actifs, le liquide ayant des caractéristiques (viscosité, tension superficielle, concentration...) qui permettent d'ajuster sa vitesse de diffusion et pouvant être introduit dans les porosités de l'objet par tout moyen comme le trempé , la pulvérisation , l'essuyage, ces procédés pouvant être éventuellement mis en oeuvre à des pressions et températures différentes des conditions standard (# 20° C, 1 atm) afin de faciliter la pénétration par absorption et / ou capillarité, et on traite de la manière décrite ci dessus l'ensemble de l'élément ou certaines parties seulement, en utilisant les techniques de la plasturgie pour la réalisation d'objets multimatières.

15. Procédé selon l'une quelconque des revendications 8 à 14 **caractérisé en ce que** on remplit les capillaires (des fibres incorporées) avec un fluide dont la viscosité ou la tension superficielle ou tout autre paramètre interdise sa diffusion ultérieure.

16. Matériaux **caractérisés en ce que** ils ont été préparés par un procédé selon l'une quelconque des revendications 8 à 15, notamment plaque de polyester insaturé contenant des fibres de chanvre à raison de 30 % en poids , la plaque ayant d'abord été plongée dans l'eau puis laissée à l'air ambiant (23 °C et 93 % HR), et **en ce qu'**on opère éventuellement une modification de la vitesse de diffusion du fluide par des altérations de la viscosité, tension superficielle, et autres paramètres connus du fluide, et / ou la fermeture de l'emballage, plusieurs paramètres de l'incorporation étant aisément modulables, notamment si l'on envisage comme principe actif un fluide à base de solvant contenant ledit principe actif :
- En variant la nature du fluide (sa viscosité ou son affinité pour la fibre entre autres) on va faire varier la vitesse de relargage.
- En faisant varier la concentration de principe actif dans la fibre, on fera varier de même la concentration en produit diffusé.
- En faisant varier la teneur en fibres du support polymère, on fera varier aussi la concentration du diffusant, mais on aura aussi une quantité de principe actif disponible plus ou moins grande.
- On peut enfin faire varier de la même manière la quantité globale de principe actif à diffuser en laissant le matériau immergé plus ou moins longtemps dans le fluide.

17. Matériaux selon l'une quelconque des revendications 1 à 7 et 16 ou préparés selon l'une quelconque des revendications 8 à 15 **caractérisés en ce que** on effectue une immersion adaptée dans un solvant neutre ce qui permet de supprimer toute trace de substance active, donc de régénérer ledit matériau, notamment un emballage, à l'état inactif et donc prêt pour une nouvelle utilisation.

18. Applications des matériaux selon l'une quelconque des revendications 1 à 7 ou 16 ou préparés selon l'une quelconque des revendications 8 à 15 ou des procédés selon l'une quelconque des revendications 8 à 16 dans les domaines suivants :
. Emballages agro-alimentaires: diffusion de produits bactéricides, fongicides, insecticides, d'agents de mûrissement, d'atmosphères protectrices, absorption ou désorption d'eau... notamment emballages avec les fonctions suivantes :
- protection contre les bactéries, les acariens, les insectes, les moisissures ...
- accélération ou ralentissement du mûrissement ...
- absorption ou émission de gaz fonctionnels ...
- maintien d'une atmosphère particulière (ex : humidité)
- absorption de l'humidité (emploi d'un superabsorbant pour mouiller les fibres), etc....
. Conditionnements industriels: relargage de produits de protection, antirouille, contrôle de l'humidité..
. Produits grand public : pièces utilitaires ou simples supports pour la diffusion d'agents antibactérien, antiacarien, antifongique..., insecticide, déodorant, désinfectant, odorant, parfum, ... , comme filtres pour aspirateurs, éléments de systèmes de climatisation et traitement de l'air du bâtiment ou des transports, poubelles ou sacs poubelle, supports olfactifs et objets de « marketing olfactif » comme des jouets olfactifs, matériaux de décoration parfumés ou de contrôle de l'humidité, ou insecticides, déodorants, anti-tabac,
. Emballages divers: contrôle de l'humidité, absorbeurs d'oxygène, diffusion de produits bactéricides, fongicides, insecticides, d'agents de mûrissement, d'atmosphères protectrice,...
